(19) Europäisches Patentamt
European Patent Office
Office européen des brevets

(11) EP 3 411 009 B1

(12) **EUROPEAN PATENT SPECIFICATION**

(45) Date of publication and mention of the grant of the patent:
**16.09.2020 Bulletin 2020/38**

(21) Application number: **17701069.1**

(22) Date of filing: **12.01.2017**

(51) Int Cl.:
***A61K 8/27*** *(2006.01)* ***A61K 8/41*** *(2006.01)*
***A61K 8/42*** *(2006.01)* ***A61K 8/49*** *(2006.01)*
***A61Q 5/00*** *(2006.01)* ***A61Q 5/12*** *(2006.01)*
***A61K 8/86*** *(2006.01)* ***A61K 8/891*** *(2006.01)*

(86) International application number:
**PCT/EP2017/050578**

(87) International publication number:
**WO 2017/133874 (10.08.2017 Gazette 2017/32)**

(54) **A HAIR CONDITIONING COMPOSITION COMPRISING POLOXAMER AND ZINC ANTI-DANDRUFF AGENT**

HAARPFLEGEZUSAMMENSETZUNG ENTHALTEND POLOXAMER UND ZINK-ANTISCHUPPENWIRKSTOFF

COMPOSITION DE CONDITIONNEMENT DES CHEVEUX COMPRENANT DU POLOXAMER ET UN AGENT ANTI-PELLICULAIRE À BASE DE ZINC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.02.2016 PCT/CN2016/073109**
**24.03.2016 PCT/EP2016/162282**

(43) Date of publication of application:
**12.12.2018 Bulletin 2018/50**

(73) Proprietors:
- **UNILEVER N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**
- **Unilever PLC**
  **London, Greater London EC4Y 0DY (GB)**
  Designated Contracting States:
  **CY GB IE MT**

(72) Inventors:
- **CAO, Qunhua**
  **Shanghai 200335 (CN)**
- **LIU, Jian**
  **Shanghai 200335 (CN)**
- **TANG, Shengnan**
  **Shanghai 200335 (CN)**
- **SUBRAMANIAN, Raghupathi**
  **Shanghai 200335 (CN)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
**WO-A1-2016/061440 US-A1- 2014 314 702**

- **DATABASE GNPD [Online] MINTEL; 1 April 2007 (2007-04-01), "Conditioner", XP002769048, retrieved from GNPD Database accession no. 687845**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 411 009 B1

## Description

### Field of the invention

The invention relates to a hair conditioning composition. The invention more particularly relates to a hair conditioning composition which comprises zinc based anti-dandruff agent and silicone conditioning agent while the composition exhibits high stability and where the silicone fouling problem is minimized to give a smooth, free flowing emulsion free of grittiness.

### Background of the invention

Hair care compositions generally provide cleansing or conditioning benefits or a combination of the two. Hair cleansing compositions generally sold as shampoos typically comprise one or more anionic cleansing surfactants which generally aid in cleaning the hair and the scalp free of undesirable soil, particles and fatty matter. Hair conditioners are another class of hair care compositions which are generally used on hair in the wet condition after the hair has been washed with a shampoo. Compositions which provide the dual benefits of shampooing and conditioning are also known.

Hair conditioners generally comprise cationic surfactants. The conditioning benefit is achieved by including one or more conditioning agents in the hair care composition. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. The most popular hair conditioning agents used are silicones.

Additionally, anti-dandruff benefit has been delivered through hair care compositions, both through shampoos and through hair conditioners. Dandruff is an issue that affects many people globally. The condition is manifested by the shedding of clumps of dead skin cells from the scalp. These are white in colour and provide an aesthetically displeasing appearance. A factor that contributes to dandruff are certain members of the *Malassezia* yeasts. To combat these, anti-dandruff products have included certain zinc salts which have anti-fungal activity, for example zinc pyrithione (ZPTO). Such a product has to perform as a hair cleansing shampoo or as a hair conditioner, while also mitigating the ill-effects of dandruff.

The present invention relates to an anti dandruff hair conditioning composition where the anti dandruff efficacy is delivered through a zinc based agent. The present inventors wish to deliver the hair conditioning benefits using a combination of a cationic surfactant and a silicone compound. They realized that when a zinc based anti-dandruff agent like ZPTO is included in a hair conditioner of this type, there is the so-called silicone fouling problem where the stability of the composition is compromised with the formation of soft grits which can foul process machinery and produce a product with lower stability. The present inventors have identified a class of polymers which could be used to minimize this silicone fouling problem.

WO13026728 (Unilever) discloses a hair conditioning composition comprising 0.1 to 10 wt% silicone and from 0.1 to 5 wt% metal pyrithione blended with non-anionic surfactant which seeks to solve the same problem of silicone fouling. The present invention has improved the technology over and above the above publication in that it is capable of minimizing silicone fouling using relatively inexpensive and widely available polymers.

DATABASE GNPD [Online] Mintel; 1 April 2007, "Conditioner", XP002769048, retrieved from GNPD Database accession no. 687845, discloses an anti-dandruff conditioner nourishes hair deeply with 1/4 moisturising cream and a ZPTO formula to treat dandruff effectively.

It is thus an object of the present invention to provide for a stable frit free hair conditioning composition that while exhibiting anti-dandruff efficacy delivers the conditioning benefits without the needed for volatile silicones.

### Summary of the invention

The present invention relates to a hair conditioning composition comprising:

(a) from 0.1 to 10% by weight of a silicone compound;
(b) from 0.1 to 5% by weight of a cationic surfactant;
(c) from 0.01 to 5% by weight of a zinc based anti dandruff agent;
(d) from 0 to 1% of a cyclomethicone compound; and
(e) a stabilizing polymer selected from a poloxamer;

wherein the silicone compound is a non-volatile silicone; and
wherein the poloxamer is a compound of the structure

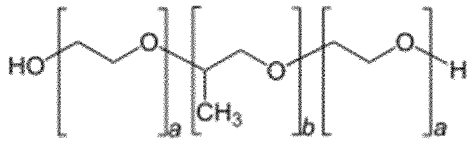

where a has a value from 2 to 130 and b has a value from 15 to 67.

## Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention, as found herein, is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'a Hair Conditioning Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or soap bar. Non-limiting examples of such compositions include leave-on hair lotions, creams, and wash-off hair conditioner or a shampoo cum conditioner, shower gels, or toilet bars especially a hair conditioning composition. The composition of the present invention is preferably a wash-off composition.

The present invention relates to a hair conditioning composition comprising:
a silicone compound; a cationic surfactant; and a zinc based anti dandruff agent; which is substantially free of cyclomethicone compound; and additionally comprises a stabilizing polymer selected from a poloxamer.

By the phrase 'substantially free of cyclomethicone is meant that it is present in an amount at the most of 1.0%, preferably at the most 0.5%, further more preferably at the most 0.1 and optimally absent in the composition. The cyclomethicone compound is preferably a volatile silicone compound. By a volatile silicone is meant a compound having the silicone structure and having a vapor pressure value at 25°C of 2.6 to 1400 Pa preferably of 13.3 Pa to 1400 Pa. Volatile silicones of the cyclomethicone type generally have a heat of vaporization value of 21 to 84 kJ/mole. Examples of cyclomethicone compounds which are substantially not present in the composition of the invention include octa methyl cyclo tetra siloxane, deca methyl cyclo penta siloxane, dodecamethyl cyclo hexa siloxane, and mixtures thereof. The most commonly used cyclomethicone compound in conventional personal care composition is deca methyl cylco penta siloxane which is popularly known as D5 and it is preferred that this compound is substantially absent in the present inventive composition.

The composition of the invention comprises a silicone compound which is not a cyclomethicone. Preferably, the composition of the invention contains emulsified droplets of a silicone conditioning agent, for enhanced conditioning performance.

The silicone compound is a non-volatile silicone. By non-volatile silicone is meant that the silicone has a vapor pressure less than 0.1 mm Hg (13.3 Pa), preferably less than 0.02 mm Hg, more preferably less than 0.01 mm Hg at 25 °C and one atmospheric pressure.

Suitable silicone compounds include polydiorganosiloxanes, in particular polydimethylsiloxanes which have the CTFA designation dimethicone. Also suitable for use in compositions of the invention are polydimethyl siloxanes having hydroxyl end groups, which have the CTFA designation dimethiconol. Also suitable for use in compositions of the invention are silicone gums having a slight degree of cross-linking, as are described for example in WO 96/31188 (UNILEVER).

The viscosity of a preferred silicone compound used as an emulsified silicone itself is typically at least 10,000 cSt at 25 °C. The viscosity of the silicone itself is preferably at least 60,000 cSt, most preferably at least 500,000 cSt, ideally at least 1,000,000 cSt. Preferably the viscosity does not exceed $10^9$ cSt for ease of formulation.

Viscosity of silicone can be determined, for example, by the relevant international standard, such as ISO 3104.

Emulsified silicones for use in the compositions of the invention will typically have a size in the composition of less than 30, preferably less than 20, more preferably less than 15 μm. Preferably the average silicone droplet is greater than 0.5 μm, more preferably greater than 1 μm, ideally from 2 to 8 μm.

A further preferred class of silicones for inclusion in the conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone". Specific examples of amino functional silicones suitable for use in the invention are the aminosilicone oils DC2-8220, DC2-8166 and DC2-8566 (all ex Dow Corning).

The total amount of silicone compound is 0.1 to 10 wt%, preferably from 0.5 wt% to 5 wt%, more preferably 0.5 wt% to 3 wt% of the composition.

The hair conditioning composition comprises conditioning surfactants selected from cationic surfactants, used singly or in admixture. Preferably, the cationic surfactants have the formula $N^+R^1R^2R^3R^4$ wherein $R^1$, $R^2$, $R^3$ and $R^4$ are independently ($C_1$ to $C_{30}$) alkyl or benzyl. Preferably, one, two or three of $R^1$, $R^2$, $R^3$ and $R^4$ are independently ($C_4$ to $C_{30}$) alkyl and the other $R^1$, $R^2$, $R^3$ and $R^4$ group or groups are ($C_1$-$C_6$) alkyl or benzyl. More preferably, one or two of $R^1$, $R^2$, $R^3$ and $R^4$ are independently ($C_6$ to $C_{30}$) alkyl and the other $R^1$, $R^2$, $R^3$ and $R^4$ groups are ($C_1$-$C_6$) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will range from 0.1 % to 5%, preferably 0.5 to 2.5% by weight of the composition.

The zinc based anti-dandruff agent as per the invention is preferably zinc pyrthione. Zinc pyrithione belongs to the class of insoluble metal pyrithione which may be represented by the following general formula:

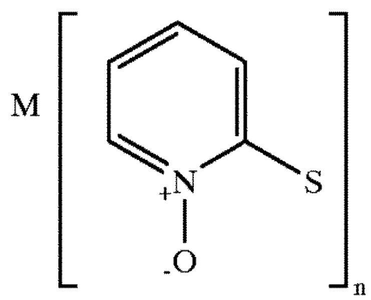

in which M is a polyvalent metal ion and n corresponds to the valency of M. In the present invention M corresponds to Zinc and n has the value of 2.

The zinc pyrithione may have any particle form suitable for use in a composition for topical application. For example, the zinc pyrithione may be in the form of amorphous or crystalline particles having a range of different particle

sizes. The zinc pyrithione may, for example, be in the form of particles having a size distribution in which at least about 90% of the particles have a size of up to 100 microns, more preferably up to 50 microns, even more preferably up to 10 microns, most preferably 5 microns or less.

Various methods for producing fine particles of metal pyrithione are described, for example, in EP-A-0 173 259. Suitable methods for determining particle size are described in that document. The insoluble metal pyrithione may be made up of one particulate form or two or more different particulate forms.

Other suitable particulate forms for the zinc pyrithione include platelets and needle-shaped particles. Platelets of zinc pyrithione are described in EP-A-0034385. The needleshaped particles are preferably of the type described in WO99/66886. For needle-shaped particles preferably at least 50% by number of the particles are needle-shaped particles having a length of between 1 μm and 50 μm.

The amount of metal pyrithione incorporated into the compositions may depend on the type of composition and the exact nature of the material used. A preferred amount of pyrithione is from about 0.01% to about 1.5% by weight of the total composition, more preferably from about 0.05% to about 1.5% by weight of the total composition.

The hair conditioning composition of the invention comprises a stabilizing polymer selected from a poloxamer.

Poloxamer is a compound of the structure

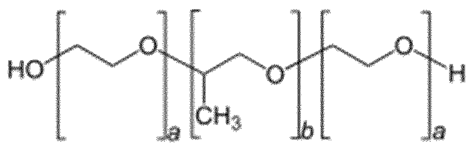

Where a has a value from 2 to 130 and b has a value from 15 to 67.

It is sold under the trade name of DC 5-7121 (Pluronic) by Dow Corning. Another poloxamer that may be used is sold under the trade name Synperonic PE/L 64 from Croda. The stabilizing polymer is preferably present in 0.01 to 0.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

The invention will now be described with reference to the following non-limiting examples.

## Examples

The following hair conditioning compositions as shown in Table - 1 were prepared.

Table - 1

| Components, wt% | Ex1 | Ex2 | Ex3 | Ex4 | Ex5 | Ex6 | Ex7 | Ex8 | Ex9 |
|---|---|---|---|---|---|---|---|---|---|
| Behentrimonium chloride | 0.70 | 0.70 | 0.70 | 0.70 | 0.70 | 0.70 | 0.70 | 0.70 | 0.70 |
| Stearamidopropyl dimethyl amine | 1.00 | 1.00 | 1.00 | 1.00 | 1.00 | 1.00 | 1.00 | 1.00 | 1.00 |
| Silicone oil | 1.20 | 1.20 | 1.20 | 1.20 | 1.20 | 1.20 | 1.20 | 1.20 | 1.20 |
| Zinc pyrithione | 0.50 | 0.50 | 0.50 | 0.50 | 0.50 | 0.50 | 0.50 | 0.50 | 0.50 |
| Cetearyl alcohol | 4.00 | 4.00 | 4.00 | 4.00 | 4.00 | 4.00 | 4.00 | 4.00 | 4.00 |
| Lactic Acid | 0.30 | 0.30 | 0.30 | 0.30 | 0.30 | 0.30 | 0.30 | 0.30 | 0.30 |

(continued)

| Components, wt% | Ex1 | Ex2 | Ex3 | Ex4 | Ex5 | Ex6 | Ex7 | Ex8 | Ex9 |
|---|---|---|---|---|---|---|---|---|---|
| Stabiliser | Cyclo Penta siloxane | - | Polox amer | Polox amer | Tinovis CD | IPM+ IHD (1: 1) | PEG 100 | Tween 20 | Dimeth iconol 5cSt |
| Stabiliser, wt% | 1.70 | - | 0.06 | 0.05 | 0.05 | 1.70 | 0.05 | 0.05 | 1.70 |
| Water | To 100 | To 100 | To 100 | To 100 | To 100 | To 100 | To 100 | To 100 | To 100 |
| % silicone fouling | 0.20 | 2.40 | 0.26 | 0.55 | 6.83 | 4.80 | 21.0 | 5.90 | 3.60 |
| Ease of cleaning | Easy | Not easy | Easy | Easy | Not easy | Difficult | Difficult | Difficult | Not easy |
| IPM+IHD: is a mixture of isopropyl myristate and isohexadecane in a weight ratio of 1:1.<br>Poloxamer: used was from DC7121 (from Dow Corning) which contains 59% silicone and 3% Poloxamer 217) | | | | | | | | | |

The compositions in Table - 1 above were subjected to a procedure that gives an estimate of the amount of gritty matter that is formed in the conditioner composition. This is measured by way of the amount of silicone that gels and sticks to the vessel, the agitator and the sieve. The %silicone fouling was measured using the following procedure.

Test procedure: About 100 g of the conditioner composition is taken in a 1000 ml beaker. About 700 g of hot water (80-90 °C) is poured into the beaker and stirred with a flat paddle at 160 rpm for 20 minutes. The diluted mass is then filtered using a 710 $\mu$m mesh. The contents of the beaker are then filled with 800 ml water (25 °C) and stirred at 160 rpm for 1 minute. The beaker, the mesh and the paddle are then dried at 45 °C for 3 hours. The increase in weight of the beaker, the paddle and the mesh is then measured which is an indication of the absolute amount of silicone fouling. The % silicone fouling is then calculated using the equation:

% silicone fouling = (absolute amount of silicone fouling) / (amount of the hair conditioner composition taken) * 100.

The silicone fouling values of the various hair conditioner compositions of Table -1 were measured and the values are summarized in Table -1. Silicone fouling higher than 2% (as per the procedure listed above) is taken as unacceptable. The information on the ease of cleaning of the reactor after the batch is completed was noted and this is summarized in Table -1. The data in Table - 1 above indicates that when polymers as per the invention is used (Examples 3 and 4) in the composition, the silicone fouling is low while several other polymers exhibit high silicone fouling and are therefore unacceptable. The reactor is also easier to clean when compositions as per the invention are used.

## Claims

**1.** A hair conditioning composition comprising:

(a) from 0.1 to 10% by weight of a silicone compound;
(b) from 0.1 to 5% by weight of a cationic surfactant;
(c) from 0.01 to 5% by weight of a zinc based anti dandruff agent;
(d) from 0 to 1% of a cyclomethicone compound; and
(e) a stabilizing polymer selected from a poloxamer

wherein the silicone compound is a non-volatile silicone: and
wherein the poloxamer is a compound of the structure

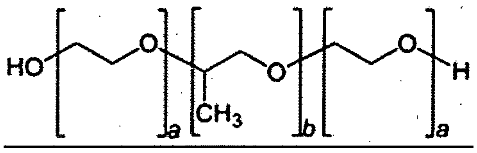

where a has a value from 2 to 130 and b has a value from 15 to 67.

2. A composition as claimed in claim 1 wherein the stabilizing polymer is present in 0.01 to 0.5% by weight of the composition.

3. A composition as claimed in any one of the preceding claims wherein the cyclomethicone compound is a volatile silicone compound.

4. A composition as claimed in any one of the preceding claims wherein the cyclomethicone compound is selected from octa methyl cyclo tetra siloxane, deca methyl cyclo penta siloxane, dodecamethyl cyclo hexa siloxane, and mixtures thereof.

5. A composition as claimed in claim 4 wherein the cylcomethicone compound is deca methyl cylco penta siloxane.

6. A composition as claimed in any one of the preceding claims wherein the cyclomethicone compound is present in less than 0.5%,

7. A composition as claimed in claim 6 wherein the cyclomethicone compound is present in less than 0.1%, preferably absent from the composition.

8. A composition as claimed in any one of the preceding claims wherein the cationic surfactant is chosen from stearamidopropyl dimethylamine, behentrimonium chloride, or stearyl trimethyl ammonium chloride.

9. A composition as claimed in any one of the preceding claims wherein the zinc based antidandruff agent is zinc pyrithione.

10. A composition as claimed in any one of the preceding claims additionally comprising 0.5 to 10 wt% of a fatty alcohol.

11. A composition as claimed in claim 10 where the fatty alcohol has a carbon chain length of 8 to 22.

**Patentansprüche**

1. Haarkonditionierzusammensetzung, umfassend:

(a) von 0,1 bis 10 Gewichts-% einer Silikonverbindung;
(b) von 0,1 bis 5 Gewichts-% eines kationischen Tensids;
(c) von 0,01 bis 5 Gewichts-% eines Antischuppenmittels auf Zinkbasis:
(d) von 0 bis 1% einer Cyclomethicon-Verbindung; und
(e) ein stabilisierendes Polymer, ausgewählt aus einem Poloxamer,

wobei die Silikonverbindung ein nicht-flüchtiges Silikon ist; und
wobei das Poloxamer eine Verbindung der Struktur

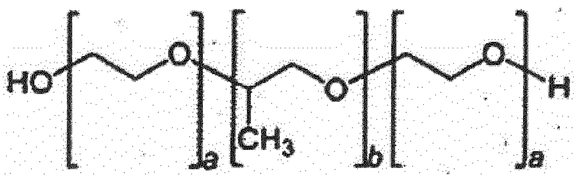

ist,

wobei a einen Wert von 2 bis 130 und b einen Wert von 15 bis 67 hat.

2. Zusammensetzung, wie im Anspruch 1 beansprucht, wobei das stabilisierende Polymer mit 0,01 bis 0,5 Gewichts-% der Zusammensetzung vorliegt.

3. Zusammensetzung, wie in irgendeinem der vorhergehenden Ansprüche beansprucht, wobei die Cyclomethiconverbindung eine flüchtige Silikonverbindung ist.

4. Zusammensetzung, wie in irgendeinem der vorhergehenden Ansprüche beansprucht, wobei die Cyclomethiconverbindung unter Octamethylcyclotetrasiloxan, Decamethylcyclopentasiloxan, Dodecamethylcyclohexasiloxan und Mischungen davon ausgewählt ist.

5. Zusammensetzung, wie im Anspruch 4 beansprucht, wobei die Cyclomethiconverbindung ein Decamethylcyclopentasiloxan ist.

6. Zusammensetzung, wie in irgendeinem der vorhergehenden Ansprüche beansprucht, wobei die Cyclomethiconverbindung mit weniger als 0,5% vorliegt.

7. Zusammensetzung, wie im Anspruch 6 beansprucht, wobei die Cyclomethiconverbindung mit weniger als 0,1% vorliegt und vorzugsweise in der Zusammensetzung fehlt.

8. Zusammensetzung, wie in irgendeinem der vorhergehenden Ansprüche beansprucht, wobei das kationische Tensid aus Stearamidopropyldimethylamin, Behentrimoniumchlorid oder Stearyltrimethylammoniumchlorid ausgewählt ist.

9. Zusammensetzung, wie in irgendeinem der vorhergehenden Ansprüche beansprucht, wobei das Antischuppenmittel auf Zinkbasis Zinkpyrithion ist.

10. Zusammensetzung, wie in irgendeinem der vorhergehenden Ansprüche beansprucht, zusätzlich umfassend 0,5 bis 10 Gew.-% eines Fettalkohols.

11. Zusammensetzung, wie im Anspruch 10 beansprucht, wobei der Fettalkohol eine Kohlenstoffkettenlänge von 8 bis 22 aufweist.

## Revendications

1. Composition de conditionnement des cheveux comprenant :

   (a) de 0,1 à 10 % en masse d'un composé de silicone ;
   (b) de 0,1 à 5 % en masse d'un tensioactif cationique ;
   (c) de 0,01 à 5 % en masse d'un agent antipelliculaire à base de zinc ;
   (d) de 0 à 1 % d'un composé de cyclométhicone ; et
   (e) un polymère stabilisant choisi parmi un poloxamère

   dans laquelle le composé de silicone est un silicone non-volatil ; et
   dans laquelle le poloxamère est un composé de la structure

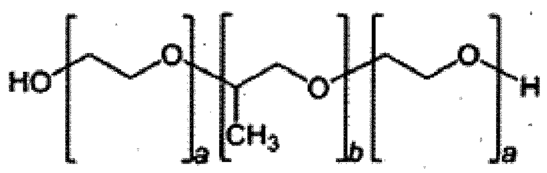

   où a présente une valeur de 2 à 130 et b présente une valeur de 15 à 67.

2. Composition selon la revendication 1, dans laquelle le polymère stabilisant est présent dans de 0,01 à 0,5 % en masse de la composition.

3. Composition selon l'une quelconque des revendications précédentes, dans laquelle le composé de cyclométhicone est un composé de silicone volatil.

4. Composition selon l'une quelconque des revendications précédentes, dans laquelle le composé de cyclométhicone est choisi parmi l'octa méthyl cyclo tétra siloxane, déca méthyl cyclo penta siloxane, dodécaméthyl cyclo hexa siloxane, et mélanges de ceux-ci.

5. Composition selon la revendication 4, dans laquelle le composé de cyclométhicone est le déca méthyl cyclo penta siloxane.

6. Composition selon l'une quelconque des revendications précédentes, dans laquelle le composé de cyclométhicone est présent dans moins de 0,5 %.

7. Composition selon la revendication 6, dans laquelle le composé de cyclométhicone est présent dans moins de 0,1 %, de préférence absent de la composition.

8. Composition selon l'une quelconque des revendications précédentes, dans laquelle le tensioactif cationique est choisi parmi la stéaramidopropyl diméthylamine, le chlorure de behentrimonium, ou le chlorure de stéaryl triméthyl ammonium.

9. Composition selon l'une quelconque des revendications précédentes, dans laquelle l'agent antipelliculaire à base de zinc est la zinc pyrithione.

10. Composition selon l'une quelconque des revendications précédentes comprenant de plus de 0,5 à 10 % en masse d'un alcool gras.

11. Composition selon la revendication 10, où l'alcool gras présente une longueur de chaîne carbonée de 8 à 22.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 13026728 A
- WO 9631188 A
- EP 0173259 A
- EP 0034385 A
- WO 9966886 A

### Non-patent literature cited in the description

- *Conditioner,* 01 April 2007